# EUROPEAN PATENT SPECIFICATION

(11) **EP 3 668 827 B1**
(45) Date of publication and mention of the grant of the patent: **23.06.2021**
(21) Application number: 18745970.6
(22) Date of filing: 02.08.2018
(51) Int. Cl.: C07C 5/48, C07C 11/04

(54) **ETHANE OXIDATIVE DEHYDROGENATION**
OXIDATIVE ETHANDEHYDRIERUNG
DÉSHYDROGÉNATION OXYDANTE D'ÉTHANE

(30) Priority: 16.08.2017 EP 17386028
(43) Date of publication of application: 24.06.2020
(73) Proprietor: Shell Internationale Research Maatschappij B.V., 2596 HR The Hague (NL)
(72) Inventor: CALVO, Laura, Mariel, 1031 HW Amsterdam (NL); ESPOSITO CASSIBBA, Ivana, Daniela, 1031 HW Amsterdam (NL); MITKIDIS, Georgios, 1031 HW Amsterdam (NL); PAJAND, Pejman, 1031 HW Amsterdam (NL); VAN ROSSUM, Guus, 1031 HW Amsterdam (NL); SAN ROMAN MACIA, Maria, Ras Laffan 3747 (QA); SCHOONEBEEK, Ronald, Jan, 1031 HW Amsterdam (NL); KLUSENER, Peter, Anton, August, 1031 HW Amsterdam (NL)
(74) Representative: Shell Legal Services IP
(86) International application number: PCT/EP2018/070953
(87) International publication number: WO 2019/034435

(56) References cited:
- WO-A1-02/24614
- US-A1- 2016 326 070

## Description

### Field of the invention

The present invention relates to a process for oxidative dehydrogenation (ODH) of ethane.

### Background of the invention

It is known to oxidatively dehydrogenate ethane in an oxidative dehydrogenation (oxydehydrogenation; ODH) process. Examples of ethane ODH processes, including catalysts and other process conditions, are for example disclosed in US7091377, WO2003064035, US20040147393, WO2010096909 and US20100256432. Mixed metal oxide catalysts containing molybdenum (Mo), vanadium (V), niobium (Nb) and optionally tellurium (Te) as the metals, can be used as ethane oxydehydrogenation catalysts.

Further, it is known to use carbon dioxide as a diluent in such ethane ODH processes. Feeding a diluent comprising carbon dioxide to an ethane ODH step is for example disclosed in US20160326070. In addition to ethylene and water, the effluent resulting from such ethane ODH step also comprises unconverted ethane and carbon dioxide. It is desired to recycle both unconverted ethane and carbon dioxide diluent to the ethane ODH step. However, a disadvantage of the process of US20160326070 (see Figures 1 to 5) is that carbon dioxide and unconverted ethane are separated from the ethane ODH effluent in two different steps. After water is removed from the ethane ODH effluent resulting from the process of Figure 1 of US20160326070, carbon dioxide is removed, by scrubbing for example, and recycled to the reactor. Finally, in a separate step of said process, ethane is separated from ethylene in a C2 splitter and the ethane is recycled to the reactor, separately from the carbon dioxide.

It is an object of the present invention to provide an ethane ODH process which comprises feeding carbon dioxide as a diluent to the ethane ODH step, in which process unconverted ethane and carbon dioxide diluent may be recycled to the ethane ODH step and ethylene product may be recovered in such way, that may be technically advantageous, simple, efficient and affordable. Such technically advantageous process would preferably result in a lower energy demand and/or lower capital expenditure.

### Summary of the invention

Surprisingly it was found that the above-mentioned object may be obtained by separating unconverted ethane and carbon dioxide diluent together, and at the same time recovering ethylene product, by means of a step which involves complexation separation and which comprises contacting at least part of a stream comprising said ethane, carbon dioxide and ethylene with a liquid solvent comprising a complexation agent, which step results in a stream comprising ethylene and a stream comprising unconverted ethane and carbon dioxide, which latter stream is subsequently recycled to the ethane ODH step.

Accordingly, the present invention relates to a process for oxidative dehydrogenation of ethane, comprising the steps of:
(a) subjecting a stream comprising ethane to oxidative dehydrogenation conditions, comprising contacting the ethane with oxygen in the presence of a catalyst comprising a mixed metal oxide, wherein a diluent comprising carbon dioxide is fed to step (a), resulting in an effluent comprising ethylene, optionally acetic acid, unconverted ethane, water, carbon dioxide, optionally unconverted oxygen, optionally carbon monoxide and optionally acetylene;
(b) removing water from at least part of the effluent resulting from step (a), resulting in a stream comprising ethylene, unconverted ethane, carbon dioxide, optionally unconverted oxygen, optionally carbon monoxide and optionally acetylene and a stream comprising water and optionally acetic acid;
(c) optionally removing unconverted oxygen and/or carbon monoxide and/or acetylene from at least part of the stream comprising ethylene, unconverted ethane, carbon dioxide, optionally unconverted oxygen, optionally carbon monoxide and optionally acetylene resulting from step (b), resulting in a stream comprising ethylene, unconverted ethane and carbon dioxide;
(d) removing ethylene from at least part of the stream comprising ethylene, unconverted ethane and carbon dioxide resulting from step (b) or (c) by a complexation separation method, which comprises contacting at least part of said stream with a liquid solvent comprising a complexation agent, resulting in a stream comprising ethylene and a stream comprising unconverted ethane and carbon dioxide;
(e) recycling at least part of the stream comprising unconverted ethane and carbon dioxide resulting from step (d) to step (a).

### Brief description of the drawings

Figure 1 depicts an embodiment covering steps (a) to (e) of the process of the present invention.
Figure 2 depicts an embodiment in relation to step (d) of the process of the present invention.

### Detailed description of the invention

The process of the present invention comprises steps (a) to (e), wherein step (c) is an optional step. These steps and optional further steps are described in further detail hereinbelow.

Thus, the process of the present invention comprises steps (a) and (b), optional step (c) and steps (d) and (e). Said process may comprise one or more intermediate steps between steps (a) and (b), between steps (b) and (c), between steps (c) and (d), and between steps (d) and (e). Further, said process may comprise one or more additional steps preceding step (a) and/or following step (e).

While the process of the present invention and a composition or stream used in said process are described in terms of "comprising", "containing" or "including" one or more various described steps and components, respectively, they can also "consist of" said one or more various described steps and components, respectively.

In the context of the present invention, in a case where a composition or stream comprises two or more components, these components are to be selected in an overall amount not to exceed 100 vol.% or 100 wt.%.

Within the present specification, "substantially no" means that no detectible amount of the component in question is present in the composition or stream.

Further, within the present specification, by "fresh ethane" reference is made to ethane which does not comprise unconverted ethane. Within the present specification, by "unconverted ethane" reference is made to ethane that was subjected to oxidative dehydrogenation conditions in step (a) of the process of the present invention, but which was not converted.

### Step (a)

Step (a) of the present process comprises subjecting a stream comprising ethane to oxidative dehydrogenation (ODH) conditions, comprising contacting the ethane with oxygen (O₂) in the presence of a catalyst comprising a mixed metal oxide, wherein a diluent comprising carbon dioxide is fed to step (a), resulting in an effluent comprising ethylene, optionally acetic acid, unconverted ethane, water, carbon dioxide, optionally unconverted oxygen, optionally carbon monoxide and optionally acetylene.

In ethane ODH step (a), ethylene is produced by oxidative dehydrogenation of ethane. Ethylene is initially formed. However, in said same step, ethylene may be oxidized into acetic acid. Further, in said same step, ethylene may be dehydrogenated into acetylene (ethyne). Ethane may also be directly converted into acetic acid or acetylene. Still further, in said same step, carbon monoxide (CO) and carbon dioxide (CO₂) may be produced, for example by combustion of ethane and/or ethylene and/or acetic acid and/or acetylene.

In ethane ODH step (a), ethane, oxygen (O₂) and carbon dioxide (CO₂) may be fed to a reactor. Said components may be fed to the reactor together or separately. That is to say, one or more feed streams, suitably gas streams, comprising one or more of said components may be fed to the reactor. For example, one feed stream comprising oxygen, ethane and carbon dioxide may be fed to the reactor. Alternatively, two or more feed streams, suitably gas streams, may be fed to the reactor, which feed streams may form a combined stream inside the reactor. For example, one feed stream comprising oxygen, another feed stream comprising ethane and still another feed stream comprising carbon dioxide may be fed to the reactor separately. In ethane ODH step (a), ethane, oxygen and carbon dioxide are suitably fed to a reactor in the gas phase.

Preferably, in ethane ODH step (a), that is to say during contacting ethane with oxygen in the presence of a catalyst, the temperature is of from 300 to 500 °C. More preferably, said temperature is of from 310 to 450 °C, more preferably of from 320 to 420 °C, most preferably of from 330 to 420 °C.

Still further, in ethane ODH step (a), that is to say during contacting ethane with oxygen in the presence of a catalyst, typical pressures are 0.1-30 or 0.1-20 bara (i.e. "bar absolute"). Further, preferably, said pressure is of from 0.1 to 15 bara, more preferably of from 1 to 10 bara, most preferably of from 3 to 10 bara. Said pressure refers to total pressure.

In addition to oxygen and ethane, carbon dioxide is also fed to ethane ODH step (a), as a diluent. One or more additional diluents, selected from the group consisting of the noble gases, nitrogen (N₂), steam (H₂O) and methane, suitably nitrogen and methane, may be fed to ethane ODH step (a). However, since in the present process carbon dioxide is already fed as a diluent to ethane ODH step (a), there is no need to add any additional diluent. Therefore, suitably, no additional diluent, in particular no steam, is fed to ethane ODH step (a). Some methane may be fed to step (a) as an impurity in the ethane feed to step (a). Further, some nitrogen may be fed to step (a) as an impurity in the oxygen feed to step (a). In these cases, methane and nitrogen function as additional diluent, in addition to carbon dioxide.

Generally, the proportion of the overall feed stream to step (a) which is attributable to a diluent is in the range from 5 to 90 vol.%, preferably from 25 to 75 vol.%. Preferably, in the case of an isothermally operated reactor, the proportion of the overall feed stream to step (a) which is attributable to a diluent is in the range from 5 to 90 vol.%, preferably from 25 to 75 vol.% and more preferably from 40 to 60 vol.%. Further, preferably, in the case of an adiabatically operated reactor, the proportion of the overall feed stream to step (a) which is attributable to a diluent is in the range from 50 to 95 vol.%, preferably from 60 to 90 vol.% and more preferably from 70 to 85 vol.%.

Preferably, the diluent as fed to step (a) comprises from 1 to 100 vol.%, more preferably 5 to 100 vol.%, more preferably 10 to 100 vol.%, more preferably 20 to 100 vol.%, more preferably 40 to 100 vol.%, more preferably 60 to 100 vol.%, more preferably 80 to 100 vol.%, more preferably 90 to 100 vol.%, more preferably 95 to 100 vol.%, and most preferably 99 to 100 vol.% of carbon dioxide, the balance consisting of one or more other diluents, selected from the group consisting of the noble gases, nitrogen (N₂), steam (H₂O) and methane, suitably nitrogen and methane. Diluents other than carbon dioxide may be used in any desired ratio relative to each other. When one or more of said additional diluents other than carbon dioxide are fed to step (a), the upper limit for the proportion of carbon dioxide in the diluent may be 20 vol.%, preferably 40 vol.%, more preferably 60 vol.%, more preferably 80 vol.%, more preferably 90 vol.%, more preferably 95 vol.%, and most preferably 99 vol.%.

The oxygen as fed to ethane ODH step (a) is an oxidizing agent, thereby resulting in oxidative dehydrogenation of ethane. Said oxygen may originate from any source, such as for example air. Suitable ranges for the molar ratio of oxygen to ethane cover ratios below, at and above the stoichiometric molar ratio (which is 0.5 for the ethane ODH reaction), suitably of from 0.01 to 1.1, more suitably of from 0.01 to 1, more suitably of from 0.05 to 0.8, most suitably of from 0.05 to 0.7. In one embodiment, the molar ratio of oxygen to ethane is of from 0.05 to 0.5, more suitably of from 0.05 to 0.47, most suitably of from 0.1 to 0.45. Further, in another embodiment, the molar ratio of oxygen to ethane is of from 0.5 to 1.1, more suitably of from 0.53 to 1, most suitably of from 0.55 to 0.9. Said ratio of oxygen to ethane is the ratio before oxygen and ethane are contacted with the catalyst. In other words, said ratio of oxygen to ethane is the ratio of oxygen as fed to ethane as fed. Obviously, after contact with the catalyst, at least part of the oxygen and ethane gets consumed. Further, said "ethane" in said molar ratio of oxygen to ethane comprises both fresh ethane and recycled (unconverted) ethane.

Preferably, pure or substantially pure oxygen (O₂) is used as oxidizing agent in step (a) of the process of the present invention. Within the present specification, by "pure or substantially pure oxygen" reference is made to oxygen that may contain a relatively small amount of one or more contaminants, including for example nitrogen (N₂), which latter amount may be at most 1 vol.%, suitably at most 7,000 parts per million by volume (ppmv), more suitably at most 5,000 ppmv, more suitably at most 3,000 ppmv, more suitably at most 1,000 ppmv, more suitably at most 500 ppmv, more suitably at most 300 ppmv, more suitably at most 200 ppmv, more suitably at most 100 ppmv, more suitably at most 50 ppmv, more suitably at most 30 ppmv, most suitably at most 10 ppmv.

Alternatively, however, it is also possible to use air or oxygen-enriched air as oxidizing agent in step (a). Such air or oxygen-enriched air would still comprise nitrogen (N₂), in an amount exceeding 1 vol.% up to 78 vol.% (air), suitably of from 1 to 50% vol.%, more suitably 1 to 30 vol.%, more suitably 1 to 20 vol.%, more suitably 1 to 10 vol.%, most suitably 1 to 5 vol.%. Said nitrogen would function as additional diluent, in addition to carbon dioxide, and would end up in the stream comprising unconverted ethane and carbon dioxide resulting from complexation separation step (d) of the present process, at least part of which stream is recycled to ethane ODH step (a) of the present process.

In order to prevent a build-up of nitrogen in the present process, nitrogen may be removed from the above-mentioned stream resulting from step (d) before recycling in step (e), for example by means of cryogenic distillation which is cumbersome. Further, said build-up may be prevented by purging part of the stream comprising unconverted ethane and carbon dioxide resulting from step (d) before the recycle, as further described below. However, by purging a part of said stream, a part of unconverted ethane is lost and not recycled to step (a). Therefore, because carbon dioxide is used as a diluent which is recycled in the present process, the above-described pure or substantially pure oxygen is preferably used as oxidizing agent in step (a) of the process of the present invention. However, in case the oxygen feed to step (a) still comprises a relatively small amount of nitrogen, such small amount of nitrogen may still be purged, before the recycle in step (e), together with additional carbon dioxide resulting from carbon dioxide production in step (a) and possibly in optional step (c).

In step (a), the ethane ODH catalyst is a catalyst comprising a mixed metal oxide. Preferably, the ODH catalyst is a heterogeneous catalyst. Further, preferably, the ODH catalyst is a mixed metal oxide catalyst containing molybdenum, vanadium, niobium and optionally tellurium as the metals, which catalyst may have the following formula:

Mo₁VₐTe_{b}Nb_{c}Oₙ

wherein:
a, b, c and n represent the ratio of the molar amount of the element in question to the molar amount of molybdenum (Mo) ;
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen.

The amount of the catalyst in ethane ODH step (a) is not essential. Preferably, a catalytically effective amount of the catalyst is used, that is to say an amount sufficient to promote the ethane oxydehydrogenation reaction.

The ODH reactor that may be used in ethane ODH step (a) may be any reactor, including fixed-bed and fluidized-bed reactors. Suitably, the reactor is a fixed-bed reactor.

Examples of oxydehydrogenation processes, including catalysts and process conditions, are for example disclosed in above-mentioned US7091377, WO2003064035, US20040147393, WO2010096909 and US20100256432.

In ethane ODH step (a), water is formed which ends up in the product stream in addition to the desired ethylene product. Further, as mentioned above, acetic acid, acetylene, carbon monoxide and carbon dioxide may be formed in step (a). Further, carbon dioxide is fed as a diluent to step (a). Still further, some of the ethane is not converted in step (a) and it may be that not all of the oxygen is converted in step (a). That is to say, ethane ODH step (a) results in an effluent comprising ethylene, optionally acetic acid, unconverted ethane, water, carbon dioxide, optionally unconverted oxygen, optionally carbon monoxide and optionally acetylene.

### Step (b)

Step (b) of the present process comprises removing water from at least part of the effluent resulting from step (a), resulting in a stream comprising ethylene, unconverted ethane, carbon dioxide, optionally unconverted oxygen, optionally carbon monoxide and optionally acetylene and a stream comprising water and optionally acetic acid.

In water removal step (b), water is suitably removed by condensation. Water in the effluent resulting from step (a) may be condensed by cooling down the latter effluent to a lower temperature, for example room temperature, after which the condensed water can be separated, resulting in a liquid stream comprising condensed water.

In water removal step (b), the temperature may be of from 10 to 150 °C, for example of from 20 to 80 °C. Suitably, in said step (b), the temperature is at least 10 °C or at least 20 °C or at least 30 °C. Further suitably, in said step (b), the temperature is at most 150 °C or at most 120 °C or at most 100 °C or at most 80 °C or at most 60 °C.

Still further, in water removal step (b), typical pressures are 0.1-30 or 0.1-20 bara (i.e. "bar absolute"). Further, preferably, said pressure is of from 0.1 to 15 bara, more preferably of from 1 to 10 bara, most preferably of from 3 to 10 bara. Said pressure refers to total pressure.

In a case wherein the stream as fed to water removal step (b) additionally comprises acetic acid, said acetic acid may be removed in water removal step (b) together with the water from said stream, suitably together with the water as condensed from said stream. During or after water removal step (b), additional water may be added to facilitate the removal of any acetic acid.

Thus, water removal step (b) results in a stream comprising ethylene, unconverted ethane, carbon dioxide, optionally unconverted oxygen, optionally carbon monoxide and optionally acetylene and a stream comprising water and optionally acetic acid. The latter stream may be a liquid stream comprising condensed water and optionally acetic acid.

### Optional step (c)

Optional step (c) of the present process comprises optionally removing unconverted oxygen and/or carbon monoxide and/or acetylene from at least part of the stream comprising ethylene, unconverted ethane, carbon dioxide, optionally unconverted oxygen, optionally carbon monoxide and optionally acetylene resulting from step (b), resulting in a stream comprising ethylene, unconverted ethane and carbon dioxide.

In case the stream comprising ethylene, unconverted ethane and carbon dioxide resulting from step (b) additionally comprises unconverted oxygen and/or carbon monoxide and/or acetylene, these additional components may be removed in optional step (c) before complexation separation step (d). Alternatively, these additional components may be removed during and/or after complexation separation step (d), as further decribed below. However, it is preferred to remove these additional components before complexation separation step (d), to prevent any difficulties in removing these during and/or after complexation separation. For example, acetylene may form a strong bond with the complexation agent in step (d). Thus, by removal of any acetylene in optional step (c), potential problems associated with the presence of acetylene in step (d) may advantageously be prevented. Likewise, in addition to the desired ethylene product, also carbon monoxide may complex to the complexation agent in step (d). Carbon monoxide complexes strongly to Cu(I) that may be present in the complexation agent used in step (d). Finally, oxygen may oxidize the metal, for example Cu(I), from a metal salt or metal complex that may be used as complexation agent in step (d). Therefore, the removal of any unconverted oxygen and/or carbon monoxide before complexation separation step (d) is also preferred.

In optional step (c) of the present process, any acetylene may be removed in any known way. For example, acetylene may be removed by selective hydrogenation or by an absorption process that uses acetone or dimethylformamide. Hydrogen (H₂) is a hydrogenation agent which may be used to hydrogenate acetylene into ethylene. Further, preferably, a selective acetylene hydrogenation catalyst is used that favours catalyzing the hydrogenation of acetylene to ethylene over the hydrogenation of ethylene to ethane.

Further, in optional step (c) of the present process, any unconverted oxygen and/or carbon monoxide may also be removed in any known way. For example, unconverted oxygen and carbon monoxide may be removed by catalytic oxidation of carbon monoxide into carbon dioxide, wherein suitably a platinum or palladium containing oxidation catalyst is used (see for example above-mentioned US20160326070). Suitably, in a case where both acetylene and unconverted oxygen and carbon monoxide are removed in optional step (c), this may be done by performing first the above-described selective hydrogenation of acetylene using hydrogen as a hydrogenation agent, followed by the above-described oxidation of carbon monoxide into carbon dioxide, so that any residual hydrogen may react with oxygen into water.

Alternatively, in optional step (c) unconverted oxygen and carbon monoxide may first be removed by distillation, for example by cryogenic distillation, followed by the above-described selective hydrogenation of acetylene using hydrogen as a hydrogenation agent. Further, it is possible to first perform the above-described selective hydrogenation of acetylene using hydrogen as a hydrogenation agent, followed by said distillation to remove unconverted oxygen, carbon monoxide and any residual hydrogen.

However, in the above-described cases it is cumbersome having to apply multiple steps to remove unconverted oxygen, carbon monoxide and acetylene before complexation separation step (d). It has been found that in one embodiment of optional step (c) of the present process, in a case where the stream comprising ethylene, unconverted ethane and carbon dioxide resulting from step (b) additionally comprises unconverted oxygen, carbon monoxide and optionally acetylene, these additional components are preferably removed advantageously in one step by oxidation of carbon monoxide and any acetylene into carbon dioxide. Thus, in said preferred embodiment, optional step (c) comprises optionally removing unconverted oxygen, carbon monoxide and optionally acetylene from at least part of the stream comprising ethylene, unconverted ethane, carbon dioxide, unconverted oxygen, carbon monoxide and optionally acetylene resulting from step (b), wherein carbon monoxide and optionally acetylene are oxidized into carbon dioxide, resulting in a stream comprising ethylene, unconverted ethane and carbon dioxide. Like with any oxidation of hydrocarbons, like acetylene, in said preferred embodiment water is produced in case acetylene is present.

In said preferred embodiment of optional step (c), unconverted oxygen may advantageously be used to oxidize both carbon monoxide and acetylene into carbon dioxide. There would be no need to add additional oxidizing agent or any other chemical, like hydrogen which can be used as a hydrogenating agent to hydrogenate acetylene, as described above. Furthermore, in said prefered embodiment, there is neither any need to apply a cumbersome (cryogenic) distillation step to remove unonverted oxygen, carbon monoxide and any hydrogen.

In said preferred embodiment of optional step (c), the temperature may vary within wide ranges and is generally of from 20 to 500 °C, and may be of from 50 to 500 °C or of from 100 to 400 °C. Preferably, in said step (c), the temperature is in the range of from 100 to 400 °C, more preferably 150 to 300 °C, more preferably 170 to 260 °C, most preferably 200 to 260 °C. In said step (c), the temperature may be at least 20 °C or at least 50 °C or at least 100 °C or at least higher than 100 °C or at least 110 °C or at least higher than 110 °C or at least 120 °C or at least higher than 120 °C or at least 130 °C or at least higher than 130 °C or at least 140 °C or at least higher than 140 °C or at least 150 °C or at least higher than 150 °C or at least 160 °C or at least higher than 160 °C or at least 170 °C or at least higher than 170 °C or at least 180 °C or at least higher than 180 °C or at least 190 °C or at least higher than 190 °C or at least 200 °C or at least higher than 200 °C or at least 210 °C or at least 220 °C or at least 230 °C or at least 240 °C. Further, in said step (c), the temperature may be at most 500 °C or at most 400 °C or at most 350 °C or at most 340 °C or at most 330 °C or at most 320 °C or at most 310 °C or at most 300 °C or at most 290 °C or at most 280 °C or at most 270 °C or at most 260 °C or at most 250 °C.

Still further, in said preferred embodiment of optional step (c), typical pressures are 0.1-30 or 0.1-20 bara (i.e. "bar absolute"). Further, preferably, said pressure is of from 0.1 to 15 bara, more preferably of from 1 to 8 bara, most preferably of from 2 to 7 bara. Said pressure refers to total pressure.

Further, in said preferred embodiment of optional step (c), additional oxygen may be fed to said step (c). Such additional oxygen is added in addition to the oxygen from the stream comprising ethylene, unconverted ethane, carbon dioxide, unconverted oxygen, carbon monoxide and optionally acetylene that is fed to said step (c). Such additional oxygen may be needed in a case where the latter stream does not contain sufficient unconverted oxygen to oxidize all of the carbon monoxide and any acetylene from the same stream into carbon dioxide. Such additional oxygen may be added either directly or indirectly to said step (c), in particular at any point before and/or during said step (c).

In said preferred embodiment of optional step (c), oxygen, carbon monoxide and optionally acetylene are removed from the stream comprising ethylene, unconverted ethane, carbon dioxide, unconverted oxygen, carbon monoxide and optionally acetylene by oxidation of carbon monoxide and any acetylene into carbon dioxide. That is to say, unconverted oxygen from the latter stream is used to oxidize carbon monoxide and any acetylene into carbon dioxide. As mentioned above, additional oxygen may be fed to fully convert all carbon monoxide and acetylene (if any) into carbon dioxide. Such oxidation may also be referred to as combustion. Thus, said step (c) results in a stream comprising ethylene, unconverted ethane and carbon dioxide.

It is also envisaged that in a case where acetylene is produced in ethane ODH step (a), such acetylene may be removed as part of said preferred embodiment of optional step (c), after water removal step (b) but before the above-described oxidation step, in particular by means of hydrogenation of acetylene into ethylene, as described above.

Suitably, in said preferred embodiment of optional step (c), oxygen may be removed to such an extent that the stream resulting from said step (c) comprises no oxygen or a residual amount of oxygen which is at most 10,000 parts per million by volume (ppmv) or at most 1,000 ppmv or at most 500 ppmv or at most 100 ppmv or at most 50 ppmv or at most 10 ppmv or at most 2 ppmv or at most 1 ppmv, based on the total volume of the stream resulting from said step (c). Further, suitably, in said preferred embodiment of optional step (c), carbon monoxide and any acetylene may be removed to such an extent that the stream resulting from said step (c) comprises no carbon monoxide and acetylene or a residual amount of carbon monoxide and acetylene which is at most 15 vol.% or at most 10 vol.% or at most 5 vol.% or at most 1 vol.% or at most 500 parts per million by volume (ppmv) or at most 100 ppmv or at most 50 ppmv or at most 10 ppmv or at most 2 ppmv or at most 1 ppmv, based on the total volume of the stream resulting from said step (c).

Said preferred embodiment of optional step (c) may be carried out in the presence of a catalyst, suitably an oxidation catalyst. Suitably, said oxidation catalyst catalyzes the oxidation of carbon monoxide and any acetylene into carbon dioxide. In particular, suitably, said oxidation catalyst catalyzes the conversion of carbon monoxide and any acetylene and oxygen into carbon dioxide by means of oxidation of carbon monoxide and any acetylene into carbon dioxide.

In said preferred embodiment of optional step (c), any oxidation catalyst that catalyzes the oxidation of carbon monoxide into carbon dioxide may be used. For example, one of the carbon monoxide oxidation catalysts as described in EP499402A1, US4956330, EP306945A1, EP421169A1, US5157204 and US5446232 may be used in said step (c). Preferably, said catalyst also catalyzes the oxidation of any acetylene into carbon dioxide.

Preferably, the above-mentioned oxidation catalyst that may be used in said preferred embodiment of optional step (c) comprises a transition metal. More preferably, said catalyst comprises one or more metals selected from the group consisting of nickel (Ni), copper (Cu), zinc (Zn), palladium (Pd), silver (Ag), platinum (Pt), gold (Au), iron (Fe), manganese (Mn), cerium (Ce), tin (Sn), ruthenium (Ru) and chromium (Cr), more preferably one or more metals selected from the group consisting of nickel, copper, zinc, platinum and ruthenium, even more preferably one or more metals selected from the group consisting of nickel, copper and zinc. Most preferably, said catalyst comprises copper and/or platinum. Suitably, said catalyst comprises copper or platinum, more suitably copper. For example, said catalyst may comprise copper and zinc. In particular, said catalyst may be a metal oxide catalyst, which may be a partially reduced metal oxide catalyst, wherein the metal(s) is (are) as described above, for example a catalyst comprising copper oxide and optionally zinc oxide. The catalyst may be a supported catalyst, wherein one or more of said metals are carried by a support, or an unsupported catalyst. In case the catalyst is a supported catalyst, the support may be any support, for example alumina, titania, silica, zirconia or silicon carbide, suitably alumina. Further, the supported catalyst may be shaped into any shape, including tablets and extrudates, or coated on a substrate.

In some cases, in said preferred embodiment of optional step (c), it may not be possible or desired to completely remove oxygen, carbon monoxide and optionally acetylene by oxidation of carbon monoxide and optionally acetylene into carbon dioxide, using unconverted oxygen and any additional oxygen as described above. If that is the case and if it is desired to remove any remaining amount of oxygen and/or carbon monoxide and/or acetylene, after said oxidation, a further removal treatment may be carried out as part of said preferred embodiment of optional step (c). Such further removal treatment may comprise passing the stream though a guard bed comprising a sorbent (adsorbent and/or absorbent) which is capable of selectively sorbing any remaining oxygen, carbon monoxide and acetylene.

### Step (d)

Step (d) of the present process comprises removing ethylene from at least part of the stream comprising ethylene, unconverted ethane and carbon dioxide resulting from step (b) or (c) by a complexation separation method, which comprises contacting at least part of said stream with a liquid solvent comprising a complexation agent, resulting in a stream comprising ethylene and a stream comprising unconverted ethane and carbon dioxide.

In step (d) of the present process, at least part of the stream comprising ethylene, unconverted ethane and carbon dioxide resulting from step (b) or (c) is subjected to a complexation separation method. In such complexation separation method olefins (ethylene) may be selectively removed from non-olefins (unconverted ethane). In the present invention, advantageously, ethylene is not only selectively separated from unconverted ethane by the complexation separation method, but also from carbon dioxide diluent which may be present in a relatively large amount and which diluent also needs to be recycled, just like unconverted ethane. In the feed to step (d) of the present process, the amount of carbon dioxide, based on the total amount of ethylene, unconverted ethane and carbon dioxide, may be of from 1 to 99 vol.%, preferably of from 5 to 95 vol.%, more preferably of from 10 to 90 vol.%, more preferably of from 20 to 85 vol.%, more preferably of from 30 to 80 vol.%, more preferably of from 40 to 75 vol.%, most preferably of from 50 to 70 vol.%. Further, said amount of carbon dioxide may be at least 1 vol.% or at least 5 vol.% or at least 10 vol.% or at least 20 vol.% or at least 30 vol.% or at least 40 vol.% or at least 50 vol.%. Still further, said amount of carbon dioxide may be at most 99 vol.% or at most 95 vol.% or at most 90 vol.% or at most 85 vol.% or at most 80 vol.% or at most 75 vol.% or at most 70 vol.%.

In the present invention, the above-mentioned complexation separation method comprises contacting at least part of the stream comprising ethylene, unconverted ethane and carbon dioxide resulting from step (b) or (c) with a liquid solvent comprising a complexation agent. The complexation agent is dissolved in said liquid solvent. That is to say, the complexation separation method in step (d) of the present process comprises so-called absorption complexation separation. In such absorption complexation separation, ethylene is preferentially complexed to the complexation agent that is dissolved in the liquid solvent.

Generally, complexation separation of olefins uses a complexation agent to selectively form a reversible complex, preferably a π-bond complex, with the olefins:

Olefin + Complexation agent ↔ Olefin-Agent Complex

Reversibility of the complexation reaction allows the olefin to be captured and released by shifting the direction of the reaction equilibrium. The forward complexation reaction is favoured by higher olefin partial pressure and lower temperature, whereas the reverse desorption reaction is favoured by lower olefin partial pressure and higher temperature. Therefore, a complexation/desorption cycle can be generated by swinging the pressure, the temperature, or both.

Preferably, in the present invention, complexation separation step (d) comprises the following cycle of substeps:
(d1) contacting at least part of the stream comprising ethylene, unconverted ethane and carbon dioxide resulting from step (b) or (c) with the liquid solvent comprising the complexation agent, resulting in a stream comprising unconverted ethane and carbon dioxide, at least part of which stream is recycled in step (e) to step (a), and a liquid stream comprising solvent, complexation agent and complexed ethylene; and
(d2) desorbing complexed ethylene from at least part of the liquid stream comprising solvent, complexation agent and complexed ethylene resulting from step (d1), resulting in a stream comprising desorbed ethylene and a liquid stream comprising solvent and complexation agent; and
(d3) recycling at least part of the liquid stream comprising solvent and complexation agent resulting from step (d2) to step (d1).

In step (d) of the present process, a suitable complexation agent is one which selectively and reversibly forms a complex with ethylene, and not or substantially not with unconverted ethane and carbon dioxide. The complexation agent may be in the form of a metal salt or a metal complex which is soluble in the liquid solvent. Salts or compounds of silver(I) or copper(I), either by themselves or combined with another metal, such as aluminium, may be used. The complexation agent is preferably a metal salt, which further preferably contains a silver(I) ion or a copper(I) ion, more preferably a silver(I) ion. Optionally, a mixture of complexation agents may be employed, for example, a mixture of copper and silver salts.

Suitable silver(I) ion containing salts include silver nitrate, silver tetrafluoroborate, silver hexafluorosilicate, silver hydroxytrifluoroborate, silver trifluoroacetate, silver perchlorate, silver triflate (CF₃SO₂O⁻Ag⁺), and silver hexafluoroantimonate (V) (SbF₆⁻Ag⁺). Suitable copper (I) ion containing salts include cuprous nitrate; cuprous halides such as cuprous chloride; cuprous sulfate; cuprous sulfonate; cuprous carboxylates; cuprous salts of fluorocarboxylic acids, such as cuprous trifluoroacetate and cuprous perfluoroacetate; cuprous fluorinated acetylacetonate; cuprous hexafluoroacetylacetonate; cuprous dodecylbenzenesulfonate; copper-aluminium halides, such as cuprous aluminium tetrachloride; CuAlCH₃Cl₃; CuAlC₂H₅Cl₃; and cuprous aluminium cyanotrichloride. Silver nitrate is the most preferred complexation agent.

The concentration of the complexation agent in the liquid solvent should be such that substantially all complexation agent is dissolved in that solvent, which depends on the (maximum) solubility of said agent in said solvent. For example, silver nitrate has a solubility (in water) of 10.9 molar (75.4 wt.%) at 35 °C. Generally, the concentration of the complexation agent may be of from 1 to 10 molar, more suitably 1 to 8 molar, more suitably 1 to 6 molar, more suitably 2 to 5 molar, most suitably 2.5 to 4 molar.

Any suitable liquid solvent or mixture of liquid solvents may be used in step (d) to dissolve the complexation agent. Within the present specification, by "liquid solvent" reference is made to a solvent which is in the liquid state at a temperature of 25 °C and a pressure of 1,01325 bar (1 atmosphere). Preferably, said liquid solvent is water, an organic solvent, an ionic liquid or a mixture thereof. Water is most preferred.

Water may be used as a solvent for silver or copper salts whereas hydrocarbon solvents, such as aromatic solvents, may be used for salts that contain organic ligands. Water is the preferred solvent because ethane and other non-olefins such as nitrogen are exceedingly sparingly soluble in aqueous solutions. In contrast, ethane has a higher solubility in hydrocarbon solvents. Olefins, like ethylene, have sufficient solubility in water for mass transfer to the dissolved complexation agent to occur at a reasonable rate.

As mentioned above, the liquid solvent to be used for dissolving the complexation agent may be an ionic liquid. As defined by Wasserscheid and Keim in "Angewandte Chemie' 2000, 112, pages 3926-3945, ionic liquids are salts which melt at a relatively low temperature. Ionic liquids are therefore already liquid at relatively low temperatures. In addition, they are in general not combustible and have no measurable vapour pressure. Within the present specification, "ionic liquid" means a salt which has a melting point or melting range which is below 100 °C.

Ionic liquids are formed from positive ions and negative ions (cations and anions, respectively), but are overall neutral in charge. The positive and also the negative ions are predominantly monovalent, but multivalent anions and/or cations which have up to five, preferably up to four, particularly preferably up to three and particularly preferably up to two electric charges are also possible. The charges within the respective ions are either localized or delocalized.

In a case where in the present invention an ionic liquid is used to dissolve the complexation agent, said ionic liquid may comprise a cation which is an N,N'-dialkylimidazolium ion or an N-alkylpyridinium ion, preferably an N,N'-dialkylimidazolium ion.

The alkyl groups in the above-mentioned N,N'-dialkylimidazolium ion and N-alkylpyridinium ion may be C₁-C₁₀ alkyl groups, preferably C₁-C₄ alkyl groups. Examples of suitable C₁-C₁₀ alkyl groups are methyl, ethyl, propyl, isopropyl, n-butyl, sec-butyl, tert-butyl, pentyl, hexyl, heptyl, octyl, 2-ethylhexyl, 2,4,4-trimethylpentyl and decyl. Preferably, the cation for the ionic liquid is an N,N'-dialkylimidazolium ion, preferably an N,N'-dialkylimidazolium ion wherein the alkyl groups are C₁-C₁₀ alkyl groups as described hereinabove, preferably C₁-C₄ alkyl groups as described hereinabove.

A particularly preferred N,N'-dialkylimidazolium ion is 1-butyl-3-methylimidazolium ion (BMIM ion). Another particularly preferred N,N'-dialkylimidazolium ion is 1,3-dimethylimidazolium ion (DMIM ion). Yet another particularly preferred N,N'-dialkylimidazolium ion is 1-ethyl-3-methylimidazolium ion (EMIM ion).

In a case where in the present invention an ionic liquid is used to dissolve the complexation agent, said ionic liquid may comprise an anion which is selected from the group consisting of tetrafluoroborate ion (BF₄⁻), alkoxyphosphonate ions, alkylsulfonate ions, hexafluorophosphate ion (PF₆⁻) and amide ions. More preferably, said anion is selected from the group consisting of tetrafluoroborate ion, alkoxyphosphonate ions and amide ions. Most preferably, said anion is tetrafluoroborate ion.

The above-mentioned alkoxyphosphonate ion is of the formula RO-PH(=O)O⁻ wherein R is an alkyl group, preferably a C₁-C₁₀ alkyl group, more preferably a C₁-C₄ alkyl group. Examples of suitable C₁-C₁₀ alkyl groups are methyl, ethyl, propyl, isopropyl, n-butyl, sec-butyl, tert-butyl, pentyl, hexyl, heptyl, octyl, 2-ethylhexyl, 2,4,4-trimethylpentyl and decyl. A particularly preferred alkoxyphosphonate ion is methoxyphosphonate ion.

The above-mentioned alkylsulfonate ion is of the formula R-S(=O)₂O⁻ wherein R is an alkyl group, preferably a C₁-C₁₀ alkyl group, more preferably a C₁-C₄ alkyl group. Examples of suitable C₁-C₁₀ alkyl groups are methyl, ethyl, propyl, isopropyl, n-butyl, sec-butyl, tert-butyl, pentyl, hexyl, heptyl, octyl, 2-ethylhexyl, 2,4,4-trimethylpentyl and decyl.

The above-mentioned amide ion is of the formula R-N⁻-R' wherein R and R' may be the same or different and are preferably electron-withdrawing substituents. Electron-withdrawing substituents, in general, are substituents that draw electrons away from an electron rich place in a molecule, in this case from the electron rich nitrogen atom in said amide ion. Preferably, R and R' are selected from the group consisting of cyano and alkanesulfonyl.

A particularly preferred amide ion is dicyanamide ion, that is to say an ion of said formula R-N⁻-R' wherein both R and R' are cyano.

Said alkanesulfonyl substituent in said amide ion is of the formula R-S(=O)₂- wherein R is an alkyl group, preferably a C₁-C₁₂ alkyl group, more preferably a C₁-C₄ alkyl group, for example methyl, ethyl or n-butyl. Said alkyl group may be linear or branched. Further, said alkyl group may be substituted with one or more halogen atoms. Said alkanesulfonyl substituent is preferably a trihalogenmethanesulfonyl substituent which is of the formula CX₃-S(=O)₂- wherein X is a halogen atom selected from the group consisting of fluorine, chlorine, bromine and iodine. More preferably, said halogen atom is fluorine. Most preferably, said trihalogenmethanesulfonyl substituent is trifluoromethanesulfonyl (CF₃-S(=O)₂-).

In a case where in the present invention an ionic liquid is used to dissolve the complexation agent, the ionic liquid preferably comprises an N,N'-dialkylimidazolium ion as described hereinabove as the cation and a tetrafluoroborate ion as the anion. Preferably, said N,N'-dialkylimidazolium ion is 1-butyl-3-methylimidazolium ion or 1-ethyl-3-methylimidazolium ion, more preferably 1-butyl-3-methylimidazolium ion.

Generally, suitable ionic liquids which may be used to dissolve the complexation agent are disclosed in "Potential of Silver-Based Room-Temperature Ionic Liquids for Ethylene/Ethane Separation", Galan Sanchez et al., Ind. Eng. Chem. Res., 2009, 48, pages 10650-10656, in particular in Table 1 of said article. Further suitable ionic liquids are disclosed in "Olefin Paraffin Separation Using Ionic Liquids", Goodrich, Cat. Rev., 2015, 28, pages 9-13, the disclosure of which article is herein incorporated by reference. Still further suitable ionic liquids are disclosed in WO201108664, WO200359483, WO200198239 and GB2383328.

Further, it is envisaged that in complexation separation step (d) of the present process, an ionic liquid is used as the liquid solvent comprising the complexation agent. In such a case, the ionic liquid is simultaneously both said liquid solvent and said complexation agent. Preferably, such ionic liquid comprises a silver(I) ion or a copper(I) ion, more preferably a silver(I) ion. Further, the anion in such ionic liquid may be an anion as described above. Examples of such ionic liquids which can be used in such a way are silver(I) bis(trifluoromethanesulfonyl) amide which is of formula [(CF₃-S(=O)₂-)₂N]⁻Ag⁺(Ag[NTf₂]), and silver (I) tris(perfluoroethyl) trifluoro phosphate which is of formula [(CF₃CF₂)₃F₃P]⁻Ag⁺ (Ag[FAP]). These and other suitable silver(I) ion containing ionic liquids are disclosed in "Liquid silver tris(perfluoroethyl) trifluoro phosphate salts as new media for propene/propane separation", Pliquette et al., Phys. Chem. Chem. Phys., 2016, 18, pages 28242-28253. However, for these ionic liquids it is still preferred that an additional liquid solvent, for example another ionic liquid which does not have a metal ion as cation, is added for liquefying and/or diluting the metal ion containing ionic liquid.

In addition to the complexation agent, the liquid solvent may comprise a modifier or mixture of modifiers, such as an acid, a salt that does not complex olefins, an oxidizing agent, or a functional organic compound. Such modifier may be used to increase the solubility and/or stability of the complexation agent in the solvent. Suitable examples of acid modifiers are nitric acid (HNO₃) and fluoroboric acid (HBF₄). In addition, such acid modifier, especially nitric acid, may reduce the physical solubility of carbon dioxide in the liquid solvent, which advantageously simplifies the separation of carbon dioxide from ethylene in complexation separation step (d). In a case where in the present invention an ionic liquid is used to dissolve the complexation agent, it is preferred that the anion of the acid modifier (e.g. HBF₄) corresponds with that of the ionic liquid.

Silver nitrate is the most preferred complexation agent in the practice of the present invention. Silver nitrate has high solubility and is very stable in water. Further, any elemental silver that would be formed can easily be reconverted into silver nitrate, by using a small amount of nitric acid. Thus, preferably, in step (d) of the present invention, an aqueous solution is used which comprises silver nitrate as the complexation agent. The latter aqueous solution further preferably comprises nitric acid as a modifier.

In above-mentioned complexation step (d1), at least part of the stream comprising ethylene, unconverted ethane and carbon dioxide resulting from step (b) or (c) is contacted with the liquid solvent comprising the complexation agent. The ethylene partial pressure in said step (d1) may be of from 0.5 to 30 bar, more suitably of from 1 to 20 bar, most suitably of from 2 to 10 bar. The ethylene partial pressure in step (d1) may be at least about as high as the ethylene partial pressure in the stream comprising ethylene, unconverted ethane and carbon dioxide resulting from step (b) or (c), or higher. Preferably, the ethylene partial pressure is increased prior to step (d1), for example by compression in a compressor. Further, the temperature of the liquid solvent as fed to said step (d1) is preferably below 50 °C, more preferably below 40 °C, and may be of from -20 to 75 °C, more suitably of from 0 to 50 °C, most suitably of from 10 to 40 °C. During step (d1) an excessive temperature rise may be avoided by internal cooling.

Step (d1) may be carried out in a countercurrent-flow column. Preferably, at least part of the stream comprising ethylene, unconverted ethane and carbon dioxide resulting from step (b) or (c) is fed to the bottom of said column and liquid solvent comprising the complexation agent is fed to the top of said column. Said column may contain a packing or trays, preferably a packing. The ethylene recovery in complexation step (d1) is preferably above 95%, more preferably above 98%.

In above-mentioned desorption step (d2), complexed ethylene is desorbed from at least part of the liquid stream comprising solvent, complexation agent and complexed ethylene resulting from step (d1). In the present invention, desorption in step (d2) may be effected by decreasing the ethylene partial pressure or by increasing the temperature or by both. A combination of decreasing the ethylene partial pressure and increasing the temperature is preferred. The total pressure in said step (d2) may be of from 1 mbar to 5 bar, more suitably of from 1 mbar to 3 bar, most suitably of from 0.5 to 1.5 bar. Further, the temperature of the liquid stream as fed to said step (d2) may be of from 55 to 130 °C, more suitably of from 65 to 130 °C, most suitably of from 80 to 120 °C. Preferably, the temperature of said liquid stream is increased prior to feeding to step (d2), for example by heating. The ethylene recovery in desorption step (d2) is preferably above 95%, more preferably above 98%. The liquid stream comprising solvent and complexation agent resulting from desorption step (d2) is recycled to complexation step (d1), preferably after cooling.

In above-mentioned complexation step (d1), a part of the unconverted ethane and carbon dioxide may be physically absorbed (dissolved) in the liquid solvent, not complexed with the complexation agent (hereinafter referred to as "absorbed" unconverted ethane and carbon dioxide). In a case wherein the liquid stream resulting from step (d1) comprises solvent, complexation agent, complexed ethylene and absorbed unconverted ethane and carbon dioxide, it is preferred to strip away said unconverted ethane and carbon dioxide from said liquid stream before feeding it to desorption step (d2). In such case, preferably, absorbed unconverted ethane and carbon dioxide are stripped from at least part of said liquid stream by contacting with a stream comprising ethylene, resulting in a stream comprising ethylene, unconverted ethane and carbon dioxide, at least part of which stream is fed to step (d1), and a liquid stream comprising solvent, complexation agent and complexed ethylene, at least part of which liquid stream is fed to step (d2). Preferably, in said stripping step, the ethylene partial pressure and the temperature are substantially not changed, so as to avoid any premature desorption before step (d2).

The above-mentioned stripping step may be carried out in a countercurrent-flow column. Preferably, at least part of the liquid stream resulting from step (d1) is fed to the top of said column and the stripping stream comprising ethylene is fed to the bottom of said column.

If acetylene is formed in ethane ODH step (a) and is not removed in optional step (c), acetylene may be present in the feed to complexation separation step (d). As mentioned above, acetylene may form a strong bond with the complexation agent in step (d). Acetylenes that contain an active hydrogen may form silver or copper acetylide compounds that have limited solubility in aqueous solution and do not decompose during desorption, so they can accumulate until they precipitate. This consumes complexation agent and may interfere with flow and generate a safety hazard. These precipitates are susceptible to detonation, especially when dry, so precautions must be taken to deal with them effectively. One way of dealing with this is to maintain silver acetylide concentration at a safe level by using silver permanganate as an oxidant. A small sidestream may be withdrawn from the desorber and heated, for example to 75 °C, under partial vacuum. Solid silver permanganate is added to destroy the acetylide, which forms carbon dioxide and free silver ion. The resulting manganese dioxide precipitates and is filtered from solution. This recovers silver without adding a foreign ion. Data and treatment of silver acetylides are given in US4174353.

If carbon monoxide is formed in ethane ODH step (a) and is not removed in optional step (c), carbon monoxide may be present in the feed to complexation separation step (d). As mentioned above, in addition to the desired ethylene product, also carbon monoxide may complex to the complexation agent in step (d). Carbon monoxide complexes strongly to Cu(I) that may be present in the complexation agent used in step (d). In the latter case, such complexed carbon monoxide is not removed in the above-mentioned stripping step, but would be desorbed, together with the ethylene, in the above-mentioned desorption step (d2), resulting in a stream comprising both ethylene and carbon monoxide.

If not all oxygen is converted in ethane ODH step (a) and the unconverted oxygen is not removed in optional step (c), oxygen may be present in the feed to complexation separation step (d). As mentioned above, oxygen may oxidize the metal, for example Cu(I), from a metal salt or metal complex that may be used as complexation agent in step (d). Any oxygen may be removed in the above-mentioned complexation step (d1) as part of the stream comprising unconverted ethane and carbon dioxide.

Further, hydrogen (H₂) may be present in the feed to complexation separation step (d). Hydrogen can cause a gradual reduction of Ag(I) to metallic silver. It is preferred to eliminate such silver reduction to prevent silver from being lost by forming colloidal particles and by plating out on surfaces. The addition of small amounts of hydrogen peroxide coupled with a maintenance level of nitric acid in the solution may stabilize the dissolved silver against precipitation. More information on such method is given in US4174353.

### Step (e)

Step (e) of the present process comprises recycling at least part of the stream comprising unconverted ethane and carbon dioxide resulting from step (d) to step (a).

The recycle stream comprising unconverted ethane and carbon dioxide resulting from step (d) may comprise of from 5 to 90 vol.% of carbon dioxide, more suitably of from 10 to 80 vol.%, most suitably of from 20 to 70 vol.%. Further, said recycle stream may comprise of from 10 to 95 vol.% of unconverted ethane, more suitably of from 20 to 90 vol.%, most suitably of from 30 to 80 vol.%. In addition, said recycle stream may comprise methane, nitrogen, carbon monoxide and/or oxygen. Suitably, the amount of methane is less than 20 vol.%, more suitably less than 10 vol.%, more suitably less than 5 vol.%. Further, suitably, the total amount of nitrogen, carbon monoxide and/or oxygen is less than 10 vol.%, more suitably less than 5 vol.%, more suitably less than 3 vol.%. Said methane may originate from the feed of fresh ethane to ethane ODH step (a). Further, said nitrogen may originate from the feed of fresh oxygen to ethane ODH step (a).

Preferably, before recycling ethane to ODH step (a), the above-mentioned stream comprising unconverted ethane and carbon dioxide resulting from step (d) is split into at least two substreams, wherein at least one split substream is recycled to step (a) and at least one split substream is not recycled to step (a). The non-recycled substream(s) are removed from the process (purged) and may therefore be discarded. The proportion of (i) the split substream(s) recycled to step (a) to (ii) the total stream before splitting is preferably of from 80 to 99.9 vol.%, more preferably of from 85 to 99 vol.%, more preferably of from 90 to 98 vol.%, most preferably of from 90 to 95 vol.%. Further, said proportion may be at least 80 vol.% or at least 85 vol.% or at least 90 vol.% or at least 95 vol.% or at least 97 vol.% or at least 98 vol.% or at least 99 vol.% or at least 99.5 vol.%. Further, said proportion may be at most 99.9 vol.% or at most 99.7 vol.% or at most 99.5 vol.% or at most 99.3 vol.% or at most 99 vol.% or at most 98 vol.% or at most 95 vol.%. Advantageously, in a case where additional carbon dioxide is produced in ethane ODH step (a) and possibly in optional step (c), such additional carbon dioxide may be removed by splitting the recycle stream before recycling, so that the amount of carbon dioxide diluent fed to ethane ODH step (a) can be kept constant.

An additional advantage in the above-mentioned case of splitting before recycling is that, at the same time when removing additional carbon dioxide produced in step (a) and possibly in optional step (c), a build-up of certain components in the present process may also advantageously be prevented, by purging a portion of the recycle stream before recyling. Said additional components that may advantageously be purged comprise for example methane from the fresh ethane feed and nitrogen from the oxygen feed, as mentioned above. Thus, this split and purge procedure has the additional advantage that the fresh ethane feed to step (a) may contain a certain amount of methane impurity, suitably up to 5 vol.% or up to 3 vol.% or up to 2 vol.% or up to 1 vol.% or up to 5,000 parts per million by volume (ppmv) or up to 2,000 ppmv or up to 1,000 ppmv or up to 500 ppmv. Likewise, the oxygen feed to step (a) may contain a certain amount of nitrogen impurity, suitably up to 5 vol.% or up to 3 vol.% or up to 2 vol.% or up to 1 vol.% or up to 5,000 parts per million by volume (ppmv) or up to 2,000 ppmv or up to 1,000 ppmv or up to 500 ppmv. By purging part of the recycle stream to step (a), the levels of these components in step (a) may advantageously also be stabilized.

### Optional further steps

Optionally, any propane is removed from the ethane containing stream in a pre-separation step prior to feeding to step (a), for example by means of distillation. Thus, in a case where propane is present in the ethane feed, it is preferred that in a step prior to step (a) of the present process, the stream comprising ethane and propane is fed to a distillation column to obtain a stream comprising propane and a stream comprising ethane. The latter stream comprising ethane, containing no or substantially no propane, is fed to step (a) of the present process. Since no or substantially no propane is present in said step (a), no or substantially no propylene is formed by ODH from propane in step (a). This advantageously prevents a cumbersome post-separation step of removing propylene from ethylene as recovered in step (d), as both propylene and ethylene may be complexed to the complexation agent used in step (d). A pre-separation step removing propane from an ethane containing stream prior to feeding to an ethane ODH step is disclosed in WO2017072086, the disclosure of which is herein incorporated by reference.

Further, in a case where the stream comprising ethylene resulting from step (d) additionally comprises one or more contaminants selected from the group consisting of propylene, carbon monoxide, oxygen, carbon dioxide and water, this or these contaminant(s) may be removed in one or more further steps. However, such further purification is not always needed. In some cases, a crude ethylene stream could be sent, without further purification, to a unit where the ethylene is further converted. In case said contaminant(s) have to be removed, this can be done in any way known to the skilled person. Propylene may be removed by distillation. Carbon monoxide may be removed by conversion (oxidation) into carbon dioxide, for example using copper oxide as oxidation catalyst, and subsequent removal of carbon dioxide. Oxygen may be removed by using it as an oxidation agent, for example in oxidizing metallic copper. Carbon dioxide may be removed by a caustic wash. Water may be removed by drying, for example by using molecular sieves.

### Figures 1 and 2

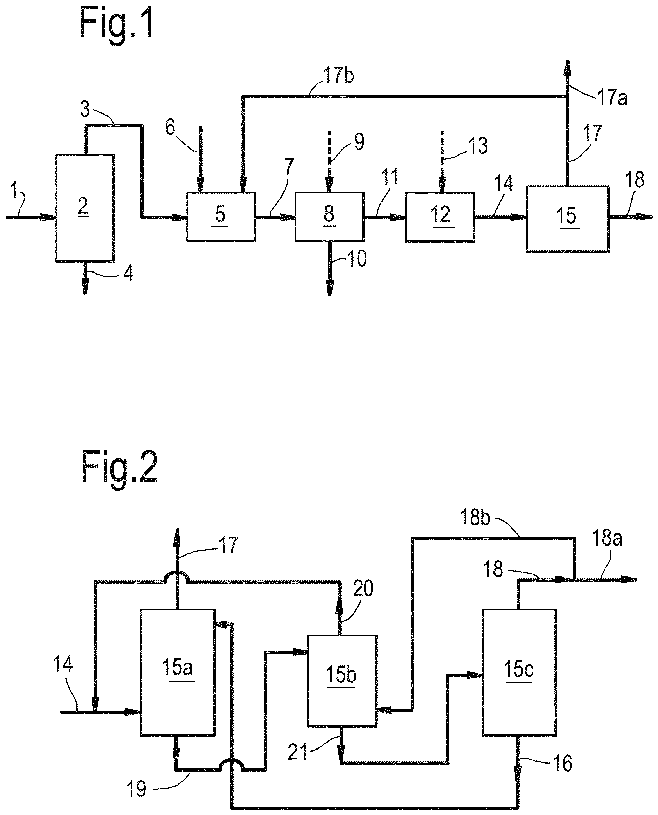

The process of the present invention is further illustrated by Figures 1 and 2.

Figure 1 depicts an embodiment covering steps (a) to (e) of the process of the present invention. In Figure 1, stream 1 comprising fresh ethane and some propane and methane is fed to distillation column 2, wherein it is separated into top stream 3 comprising fresh ethane and methane and bottom stream 4 comprising propane. Said stream 3, stream 6 comprising oxygen and recycle stream 17b comprising carbon dioxide (diluent), unconverted ethane and some methane are fed to oxidative dehydrogenation (ODH) unit 5 containing an ethane ODH catalyst comprising a mixed metal oxide and operating under ODH conditions, wherein ethane is converted into ethylene in accordance with the above-described step (a) of the process of the present invention. Product stream 7 coming from ODH unit 5 comprises water, methane, ethane, ethylene, oxygen, carbon monoxide, acetylene, carbon dioxide and acetic acid. Said stream 7 is fed to water condensation unit 8. In water condensation unit 8, water and acetic acid are removed by condensation via stream 10 in accordance with the above-described step (b) of the process of the present invention. Optionally, additional water is fed to water removal unit 8 via stream 9. Stream 11 coming from water condensation unit 8, which comprises methane, ethane, ethylene, oxygen, carbon monoxide, acetylene and carbon dioxide, is fed to gas clean-up reactor 12. In gas clean-up reactor 12, oxygen, acetylene and carbon monoxide are removed in accordance with the above-described step (c) of the process of the present invention. In particular, carbon monoxide and acetylene are oxidized into carbon dioxide, using the above-described oxidation catalyst, in particular an oxidation catalyst which comprises copper. Optionally, additional oxygen is fed to gas clean-up reactor 12 via stream 13. Product stream 14 coming from gas clean-up reactor 12 comprises methane, ethane, ethylene and carbon dioxide. Said stream 14 is fed to complexation separation unit 15. In complexation separation unit 15, a complexation separation method is applied in accordance with the above-described step (d) of the process of the present invention. Complexation separation unit 15 is further described below with reference to Figure 2. Stream 18 coming from complexation separation unit 15 comprises ethylene. Stream 17 coming from complexation separation unit 15 comprises carbon dioxide (diluent), (unconverted) ethane and some methane. Said stream 17 is split into recycle substream 17b which is fed to ODH unit 5 and substream 17a which is purged from the process. By such a purge, a build-up of methane (present in the fresh ethane feed stream 1 and in stream 3), additional carbon dioxide (as produced in ethane ODH unit 5 and gas clean-up reactor 12) and any nitrogen (which could be present in the fresh oxygen feed stream 6) is prevented.

Figure 2 depicts an embodiment in relation to step (d) of the process comprising steps (a) to (e) as depicted in Figure 1. In Figure 2, stream 14 comprising methane, ethane, ethylene and carbon dioxide, which comes from gas clean-up reactor 12, is fed to the bottom of absorber 15a which is part of complexation separation unit 15. Before feeding said stream 14 to absorber 15a, it is compressed in a compressor (not shown in Figure 1 or 2). In absorber 15a, said stream 14 is contacted with the liquid solvent comprising the complexation agent, in accordance with the above-described step (d1), which liquid solvent is fed to the top of absorber 15a via stream 16. The ethylene partial pressure in absorber 15a may be about 4 bar and the temperature of liquid stream 16 as fed to absorber 15a may be about 30 °C. Top stream 17 coming from absorber 15a comprises carbon dioxide, ethane and methane. Bottom stream 19 coming from absorber 15a is a liquid stream comprising solvent, complexation agent, complexed ethylene and absorbed methane, ethane and carbon dioxide, which stream is fed to the top of stripper 15b which is also part of complexation separation unit 15. In stripper 15b, said absorbed methane, ethane and carbon dioxide are stripped by contacting with stream 18b comprising ethylene, as described above, which stream 18b is fed to the bottom of stripper 15b. The ethylene partial pressure in stripper 15b may be about 4 bar and the temperature of liquid stream 19 as fed to stripper 15b may be about 30 °C. Top stream 20 coming from stripper 15b comprises ethylene, methane, ethane and carbon dioxide and is compressed in a compressor (not shown in Figure 2) and then fed to absorber 15a via stream 14. Bottom stream 21 coming from stripper 15b is a liquid stream which comprises solvent, complexation agent and complexed ethylene and is fed to desorber 15c which is also part of complexation separation unit 15. In desorber 15c, ethylene is desorbed in accordance with the above-described step (d2). The total pressure in desorber 15c may be about 500 mbar and the temperature of liquid stream 21 as fed to desorber 15c may be about 80 °C. Top stream 18 coming from desorber 15c comprises desorbed ethylene and is split into two substreams 18a and 18b. Substream 18b is compressed in a compressor (not shown in Figure 2) and then fed to stripper 15b. Substream 18a may be further purified. Bottom stream 16 coming from desorber 15c is a liquid stream which comprises solvent and complexation agent and is recycled to absorber 15a in accordance with the above-described step (d3).

## Claims

1. Process for oxidative dehydrogenation of ethane, comprising the steps of:
(a) subjecting a stream comprising ethane to oxidative dehydrogenation conditions, comprising contacting the ethane with oxygen in the presence of a catalyst comprising a mixed metal oxide, wherein a diluent comprising carbon dioxide is fed to step (a), resulting in an effluent comprising ethylene, optionally acetic acid, unconverted ethane, water, carbon dioxide, optionally unconverted oxygen, optionally carbon monoxide and optionally acetylene;
(b) removing water from at least part of the effluent resulting from step (a), resulting in a stream comprising ethylene, unconverted ethane, carbon dioxide, optionally unconverted oxygen, optionally carbon monoxide and optionally acetylene and a stream comprising water and optionally acetic acid;
(c) optionally removing unconverted oxygen and/or carbon monoxide and/or acetylene from at least part of the stream comprising ethylene, unconverted ethane, carbon dioxide, optionally unconverted oxygen, optionally carbon monoxide and optionally acetylene resulting from step (b), resulting in a stream comprising ethylene, unconverted ethane and carbon dioxide;
(d) removing ethylene from at least part of the stream comprising ethylene, unconverted ethane and carbon dioxide resulting from step (b) or (c) by a complexation separation method, which comprises contacting at least part of said stream with a liquid solvent comprising a complexation agent, resulting in a stream comprising ethylene and a stream comprising unconverted ethane and carbon dioxide;
(e) recycling at least part of the stream comprising unconverted ethane and carbon dioxide resulting from step (d) to step (a).

2. Process according to claim 1, wherein step (d) comprises:
(d1) contacting at least part of the stream comprising ethylene, unconverted ethane and carbon dioxide resulting from step (b) or (c) with the liquid solvent comprising the complexation agent, resulting in a stream comprising unconverted ethane and carbon dioxide, at least part of which stream is recycled in step (e) to step (a), and a liquid stream comprising solvent, complexation agent and complexed ethylene; and
(d2) desorbing complexed ethylene from at least part of the liquid stream comprising solvent, complexation agent and complexed ethylene resulting from step (d1), resulting in a stream comprising desorbed ethylene and a liquid stream comprising solvent and complexation agent; and
(d3) recycling at least part of the liquid stream comprising solvent and complexation agent resulting from step (d2) to step (d1).

3. Process according to claim 2, wherein the liquid stream resulting from step (d1) comprises solvent, complexation agent, complexed ethylene and absorbed unconverted ethane and carbon dioxide, wherein absorbed unconverted ethane and carbon dioxide are stripped from at least part of said liquid stream by contacting with a stream comprising ethylene, resulting in a stream comprising ethylene, unconverted ethane and carbon dioxide, at least part of which stream is fed to step (d1), and a liquid stream comprising solvent, complexation agent and complexed ethylene, at least part of which liquid stream is fed to step (d2).

4. Process according to any one of the preceding claims, wherein in the feed to step (d) the amount of carbon dioxide, based on the total amount of ethylene, unconverted ethane and carbon dioxide, is of from 1 to 99 vol.%, preferably of from 5 to 95 vol.%, more preferably of from 10 to 90 vol.%, more preferably of from 20 to 85 vol.%, more preferably of from 30 to 80 vol.%, more preferably of from 40 to 75 vol.%, most preferably of from 50 to 70 vol.%.

5. Process according to any one of the preceding claims, wherein the complexation agent in step (d) is a metal salt.

6. Process according to claim 5, wherein the metal salt contains a silver(I) ion or a copper(I) ion, preferably a silver(I) ion.

7. Process according to claim 6, wherein the metal salt is silver nitrate.

8. Process according to any one of the preceding claims, wherein the liquid solvent in step (d) is water, an organic solvent, an ionic liquid or a mixture thereof, preferably water.

9. Process according to any one of the preceding claims, wherein before step (e) the stream comprising unconverted ethane and carbon dioxide resulting from step (d) is split into at least two substreams, wherein at least one split substream is recycled to step (a) and at least one split substream is not recycled to step (a).

10. Process according to claim 9, wherein the proportion of (i) the split substream(s) recycled to step (a) to (ii) the total stream before splitting is of from 80 to 99.9 vol.%, more preferably of from 85 to 99 vol.%, more preferably of from 90 to 98 vol.%, most preferably of from 90 to 95 vol.%.

## Patentansprüche

1. Vorgang für eine oxidative Dehydrierung von Ethan, der die folgenden Schritte umfasst:
(a) Aussetzen eines Stroms, der Ethan umfasst, von Bedingungen der oxidativen Dehydrierung, das ein Inberührungbringen des Ethans mit Sauerstoff in der Gegenwart eines Katalysators umfasst, der ein gemischtes Metalloxid umfasst, wobei ein Verdünnungsmittel, das Kohlenstoffdioxid umfasst, zu Schritt (a) zugeführt wird, das einen Effluenten ergibt, der Ethylen, optional Essigsäure, nicht umgesetztes Ethan, Wasser, Kohlenstoffdioxid, optional nicht umgesetzten Sauerstoff, optional Kohlenstoffmonoxid und optional Acetylen umfasst;
(b) Entfernen von Wasser aus wenigstens einem Teil des Effluenten, der sich aus Schritt (a) ergibt, das einen Strom, der Ethylen, nicht umgesetztes Ethan, Kohlenstoffdioxid, optional nicht umgesetzten Sauerstoff, optional Kohlenstoffmonoxid und optional Acetylen umfasst, und einen Strom ergibt, der Wasser und optional Essigsäure umfasst;
(c) optional Entfernen von nicht umgesetztem Sauerstoff und/oder Kohlenstoffmonoxid und/oder Acetylen aus wenigstens einem Teil des Stroms, der Ethylen, nicht umgesetztes Ethan, Kohlenstoffdioxid, optional nicht umgesetzten Sauerstoff, optional Kohlenstoffmonoxid und optional Acetylen umfasst, der sich aus Schritt (b) ergibt, das einen Strom ergibt, der Ethylen, nicht umgesetztes Ethan und Kohlenstoffdioxid umfasst;
(d) Entfernen von Ethylen aus wenigstens einem Teil des Stroms, der Ethylen, nicht umgesetztes Ethan und Kohlenstoffdioxid umfasst, der sich aus Schritt (b) oder (c) ergibt, durch ein Komplexierungstrennungsverfahren, das das Inberührungbringen wenigstens eines Teils des Stroms mit einem flüssigen Lösungsmittel umfasst, das ein Komplexierungsmittel umfasst, das einen Strom, der Ethylen umfasst, und einen Strom ergibt, der nicht umgesetztes Ethan und Kohlenstoffdioxid umfasst;
(e) Rückführen wenigstens eines Teils des Stroms, der nicht umgesetztes Ethan und Kohlenstoffdioxid umfasst, der sich aus Schritt (d) ergibt, zu Schritt (a).

2. Vorgang nach Anspruch 1, wobei Schritt (d) Folgendes umfasst:
(d1) Inberührungbringen wenigstens eines Teils des Stroms, der Ethylen, nicht umgesetztes Ethan und Kohlenstoffdioxid umfasst, der sich aus Schritt (b) oder (c) ergibt, mit dem flüssigen Lösungsmittel, das das Komplexierungsmittel umfasst, das einen Strom, der nicht umgesetztes Ethan und Kohlenstoffdioxid umfasst, wobei wenigstens ein Teil des Stroms in Schritt (e) zu Schritt (a) rückgeführt wird, und einen flüssigen Strom ergibt, der Lösungsmittel, Komplexierungsmittel und komplexiertes Ethylen umfasst; und
(d2) Desorbieren von komplexiertem Ethylen aus wenigstens einem Teil des flüssigen Stroms, der Lösungsmittel, Komplexierungsmittel und komplexiertes Ethylen umfasst, der sich aus Schritt (d1) ergibt, das einen Strom, der desorbiertes Ethylen umfasst, und einen flüssigen Strom ergibt, der Lösungsmittel und Komplexierungsmittel umfasst; und
(d3) Rückführen wenigstens eines Teils des flüssigen Stroms, der Lösungsmittel und Komplexierungsmittel umfasst, der sich aus Schritt (d2) ergibt, zu Schritt (d1).

3. Vorgang nach Anspruch 2, wobei der flüssige Strom, der sich aus Schritt (d1) ergibt, Lösungsmittel, Komplexierungsmittel, komplexiertes Ethylen und absorbiertes nicht umgesetztes Ethan und Kohlenstoffdioxid umfasst, wobei absorbiertes nicht umgesetztes Ethan und Kohlenstoffdioxid von wenigstens einem Teil des flüssigen Stroms durch Inberührungbringen mit einem Strom gestrippt werden, der Ethylen umfasst, das einen Strom, der Ethylen, nicht umgesetztes Ethan und Kohlenstoffdioxid umfasst, wobei wenigstens ein Teil des Stroms zu Schritt (d1) zugeführt wird, und einen flüssigen Strom ergibt, der Lösungsmittel, Komplexierungsmittel und komplexiertes Ethylen umfasst, wobei wenigstens ein Teil des flüssigen Stroms zu Schritt (d2) zugeführt wird.

4. Vorgang nach einem der vorhergehenden Ansprüche, wobei in der Zufuhr zu Schritt (d) die Menge an Kohlenstoffdioxid, basierend auf der Gesamtmenge an Ethylen, nicht umgesetztem Ethan und Kohlenstoffdioxid, von 1 bis 99 Vol.-% beträgt, bevorzugt von 5 bis 95 Vol.-%, stärker bevorzugt von 10 bis 90 Vol.-%, stärker bevorzugt von 20 bis 85 Vol.-%, stärker bevorzugt von 30 bis 80 Vol.-%, stärker bevorzugt von 40 bis 75 Vol.-%, am stärksten bevorzugt von 50 bis 70 Vol.-%.

5. Vorgang nach einem der vorhergehenden Ansprüche, wobei das Komplexierungsmittel in Schritt (d) ein Metallsalz ist.

6. Vorgang nach Anspruch 5, wobei das Metallsalz ein Silber(I)-Ion oder ein Kupfer(I)-Ion enthält, bevorzugt ein Silber(I)-Ion.

7. Vorgang nach Anspruch 6, wobei das Metallsalz Silbernitrat ist.

8. Vorgang nach einem der vorhergehenden Ansprüche, wobei das flüssige Lösungsmittel in Schritt (d) Wasser, ein organisches Lösungsmittel, eine ionische Flüssigkeit oder eine Mischung davon ist, bevorzugt Wasser.

9. Vorgang nach einem der vorhergehenden Ansprüche, wobei vor Schritt (e) der Strom, der nicht umgesetztes Ethan und Kohlenstoffdioxid umfasst, der sich aus Schritt (d) ergibt, in wenigstens zwei Unterströme geteilt wird, wobei wenigstens ein geteilter Unterstrom zu Schritt (a) rückgeführt wird und wenigstens ein geteilter Unterstrom nicht zu Schritt (a) rückgeführt wird.

10. Vorgang nach Anspruch 9, wobei das Verhältnis von
(i) dem/den geteilten Unterstrom/Unterströmen, der/die zu Schritt (a) rückgeführt wird/werden, zu
(ii) dem Gesamtstrom vor dem Teilen von 80 bis 99,9 Vol.-% beträgt, stärker bevorzugt von 85 bis 99 Vol.-%, stärker bevorzugt von 90 bis 98 Vol.-%, am stärksten bevorzugt von 90 bis 95 Vol.-%.

## Revendications

1. Processus de déshydrogénation oxydante de l'éthane, comprenant les étapes consistant à :
(a) soumettre un courant comprenant de l'éthane à des conditions de déshydrogénation oxydante, comprenant la mise en contact de l'éthane avec de l'oxygène en présence d'un catalyseur comprenant un oxyde métallique mixte, un diluant comprenant du dioxyde de carbone étant alimenté à l'étape (a), ce qui permet d'obtenir un effluent comprenant de l'éthylène, éventuellement de l'acide acétique, de l'éthane non converti, de l'eau, le dioxyde de carbone, éventuellement de l'oxygène non converti, éventuellement du monoxyde de carbone et éventuellement l'acétylène ;
(b) éliminer de l'eau d'au moins une partie de l'effluent issu de l'étape (a), ce qui permet d'obtenir un courant comprenant de l'éthylène, de l'éthane non converti, du dioxyde de carbone, éventuellement de l'oxygène non converti, éventuellement du monoxyde de carbone et éventuellement de l'acétylène et un courant comprenant de l'eau et éventuellement de l'acide acétique ;
(c) éliminer éventuellement de l'oxygène non converti et/ou du monoxyde de carbone et/ou de l'acétylène d'au moins une partie du courant comprenant de l'éthylène, de l'éthane non converti, du dioxyde de carbone, éventuellement de l'oxygène non converti, éventuellement du monoxyde de carbone et éventuellement de l'acétylène issu de l'étape (b), ce qui permet d'obtenir un courant comprenant de l'éthylène, de l'éthane non converti et du dioxyde de carbone ;
(d) éliminer de l'éthylène d'au moins une partie du courant comprenant de l'éthylène, de l'éthane non converti et du dioxyde de carbone issu de l'étape (b) ou (c) par un procédé de séparation par complexation, qui comprend la mise en contact d'au moins une partie dudit courant avec un solvant liquide comprenant un agent de complexation, ce qui permet d'obtenir un courant comprenant de l'éthylène et un courant comprenant de l'éthane non converti et du dioxyde de carbone ;
(e) recycler au moins une partie du courant comprenant de l'éthane non converti et du dioxyde de carbone issu de l'étape (d) à l'étape (a).

2. Processus selon la revendication 1, dans lequel l'étape (d) comprend :
(d1) la mise en contact d'au moins une partie du courant comprenant de l'éthylène, de l'éthane non converti et du dioxyde de carbone issu de l'étape (b) ou (c) avec le solvant liquide comprenant l'agent de complexation, ce qui permet d'obtenir un courant comprenant de l'éthane non converti et du dioxyde de carbone, au moins une partie duquel courant étant recyclée dans l'étape (e) à l'étape (a), et un courant liquide comprenant un solvant, un agent de complexation et de l'éthylène complexé ; et
(d2) la désorption de l'éthylène complexé d'au moins une partie du courant liquide comprenant un solvant, un agent de complexation et de l'éthylène complexé issu de l'étape (d1), ce qui permet d'obtenir un courant comprenant de l'éthylène désorbé et un courant liquide comprenant un solvant et un agent de complexation ; et
(d3) le recyclage d'au moins une partie du courant liquide comprenant un solvant et un agent de complexation issu de l'étape (d2) à l'étape (d1).

3. Processus selon la revendication 2, dans lequel le courant liquide issu de l'étape (d1) comprend un solvant, un agent de complexation, de l'éthylène complexé et de l'éthane non converti et du dioxyde de carbone absorbés, de l'éthane non converti et du dioxyde de carbone absorbés étant extraits d'au moins une partie dudit courant liquide par mise en contact avec un courant comprenant de l'éthylène, ce qui permet d'obtenir un courant comprenant de l'éthylène, de l'éthane non converti et du dioxyde de carbone, au moins une partie duquel courant étant alimentée à l'étape (d1), et un courant liquide comprenant un solvant, un agent de complexation et de l'éthylène complexé, au moins une partie duquel courant de liquide étant alimentée à l'étape (d2).

4. Processus selon l'une quelconque des revendications précédentes, dans lequel, lors de l'alimentation à l'étape (d), la quantité de dioxyde de carbone, sur la base de la quantité totale d'éthylène, d'éthane non converti et de dioxyde de carbone, est de 1 à 99 % en volume, de préférence de 5 à 95 % en volume, plus préférablement de 10 à 90 % en volume, plus préférablement de 20 à 85 % en volume, plus préférablement de 30 à 80 % en volume, plus préférablement de 40 à 75 % en volume, idéalement de 50 à 70 % en volume.

5. Processus selon l'une quelconque des revendications précédentes, dans lequel l'agent de complexation à l'étape (d) est un sel métallique.

6. Processus selon la revendication 5, dans lequel le sel métallique contient un ion argent (I) ou un ion cuivre (I), de préférence un ion argent (I).

7. Processus selon la revendication 6, dans lequel le sel métallique est le nitrate d'argent.

8. Processus selon l'une quelconque des revendications précédentes, dans lequel le solvant liquide à l'étape (d) est de l'eau, un solvant organique, un liquide ionique ou leur mélange, de préférence de l'eau.

9. Processus selon l'une quelconque des revendications précédentes, dans lequel avant l'étape (e) le courant comprenant de l'éthane non converti et du dioxyde de carbone issu de l'étape (d) est divisé en au moins deux sous-courants, au moins un sous-courant divisé étant recyclé à l'étape (e) et au moins un sous-courant divisé n'étant pas recyclé à l'étape (a).

10. Processus selon la revendication 9, dans lequel la proportion (i) du ou des sous-courants divisés recyclés à l'étape (a) à (ii) le flux total avant la division est de 80 à 99,9 % en volume, plus préférablement de 85 à 99 % en volume, plus préférablement de 90 à 98 % en volume, idéalement de 90 à 95 % en volume.
